# EUROPÄISCHE PATENTSCHRIFT

(11) **EP 0 355 021 B1**
(45) Veröffentlichungstag und Bekanntmachung des Hinweises auf die Patenterteilung: **18.11.1993**
(21) Anmeldenummer: 89114893.4
(22) Anmeldetag: 11.08.1989
(51) Int. Cl.: A61M 5/142, F04B 43/12

(54) **Druckinfusionsapparat**
Pressure infusion device
Dispositif de perfusion par pression

(30) Priorität: 16.08.1988 DE 3827722
(43) Veröffentlichungstag der Anmeldung: 21.02.1990
(73) Patentinhaber: Fresenius AG, 61350 Bad Homburg (DE)
(72) Erfinder: Neuder, Klaus, Dr. Dipl.-Ing., D-6053 Obertshausen (DE)
(74) Vertreter: Fuchs Mehler Weiss

(56) Entgegenhaltungen:
- WO-A-82/00590
- DE-A- 2 028 973
- FR-A- 2 503 799
- US-A- 4 758 228

## Beschreibung

Die Erfindung betrifft einen Druckinfusionsapparat mit einem Antrieb, der über eine Fördermechanik, wie Rotor oder Fingerperistaltik, eine während der Förderung periodische, fortschreitende Druckbewegung auf einen Abschnitt eines Schlauches ausübt, in dem sich das zu fördernde Medium befindet, und mit einer während der Förderung feststehenden Andruckeinrichtung, wie Andruckplatte, Stator oder Schlauchplatte.

Druckinfusionsapparate werden eingesetzt, um Infusionsflüssigkeiten, die sich in einem Vorratsbehälter befinden, mit einer entsprechend der Therapie gewünschten Geschwindigkeit in die Vene des Patienten zu pumpen. Der Druckinfusionsapparat ist an dem Verbindungsschlauch zwischen diesem Vorratsgefäß und der Kanüle angeordnet. Druckinfusionsapparate sind in verschiedenen Ausführungen bekannt.

Eine bekannte Ausführung weist einen Stator und einen von einem Motor angetriebenen Rotor auf, wobei der Pumpschlauch zwischen Rotor und Stator eingeklemmt ist. Durch die Drehung des Rotors wird der Schlauch in Richtung zum Patienten kontinuierlich zusammengedrückt, wodurch die im Schlauch befindliche Infusionsflüssigkeit mit vorgegebener Geschwindigkeit in die Vene gepumpt wird.

Ein derartiger Druckinfusionsapparat ist beispielsweise aus der US-PS 4,184,815 bekannt. Zum

Einlegen des Schlauches muß der Rotor von Hand in eine bestimmte Position gedreht werden, damit die am Rotor angeordneten Quetschrollen das Einlegen nicht behindern. Nachdem ein Abschnitt des Schlauches zwischen Rotor und Stator plaziert worden ist, muß der Rotor von Hand weitergedreht werden, wobei bereits die erste Quetschrolle gegen den Schlauch drückt. Dazu muß vom Anwender (Arzt, Krankenschwester, Patient usw.) eine bestimmte Kraft aufgebracht werden.

Bei einer peristaltischen Pumpe, wie sie aus der US-PS 4,025,241 bekannt ist, muß zum Einlegen des Schlauchsegmentes der Stator gegen die Kraft zweier Federn vom Rotor weggerückt werden.

Aus der FR-A-25 03 799 ist eine Rollpumpe für medizinische Zwecke bekannt, bei der der Förderschlauch zwischen den Rollkörpern und einem Stützteil gequetscht wird. Zum Auswechseln des Schlauches muß das Stützteil gegen die Kraft von mehreren Federn bewegt werden. In der abgezogenen Montagestellung kann das Stützteil verriegelt werden.

Bei der aus der US-A-47 58 228 bekannten Infusionspumpe muß von Hand ein Knopf gedreht werden, um das Pumpenrad aus seiner Exzenterstellung in eine mittige Position zu bringen, damit der Schlauch eingelegt werden kann.

Gemäß der WO-A-82 00 590 ist an der Pumpe eine Andruckplatte vorgesehen, die manuell durch Drehen eines Knopfes über eine exzentrische Nocke bewegt werden kann. Eine ähnliche Einrichtung mit einer exzentrischen Nocke, die gegen die Kraft einer Vorspannfeder bewegt wird, ist aus der DE-A-20 28 973 bekannt.

Peristaltische Pumpen können auch mit einer Fingerperistaltik ausgerüstet sein, bei der eine große Anzahl von Fingern oder Pumpenstößel auf einer Geraden nebeneinander angeordnet sind. Zwischen den freien Enden der Pumpenstößel und einer Andruckplatte ist der mit dem zu fördernden Medium gefüllte Infusionsschlauch eingeklemmt. Durch eine peristaltische Bewegung der Pumpenstößel wird die im Infusionsschlauch befindliche Lösung gefördert. Auch bei dieser Ausführung muß der Anwender zum Einlegen des Schlauches die Andruckplatte gegen die Wirkung einer Feder von den Pumpenstößeln wegdrücken.

Schließlich sind noch statorlose Rollenpumpen bekannt, bei denen der Schlauch derart in einer Schlauchplatte befestigt ist, daß das Schlauchsegment eine Schlaufe bildet. Zum Einlegen des Schlauchsegments muß die Schlaufe über den Rotor gelegt und die Schlauchpumpe in eine Halterung gesteckt werden. Schlaufengröße und Abstand Rotor/Halterung sind so aufeinander abgestimmt, daß beim Einlegen der Schlauchplatte in die Halterung der Schlauch durch die Rollen des Rotors zusammengedrückt wird. Dieses Vorspannen des Schlauchsegmentes erfordert vom Anwender ebenfalls eine bestimmte Kraft.

Bei allen bekannten Druckinfusionsapparaten muß die Andruckeinrichtung für den Schlauch unter Aufwendung mechanischer Arbeit des Anwenders von der Fördermechanik abgehoben oder angedrückt werden. Dies geschieht entweder durch direktes Fassen der Andruckeinrichtung oder Fördermechanik oder durch indirektes "Fassen" mit Hilfe von Hebeln oder Knöpfen.

Es ist ebenso bekannt, einen Teil der vom Anwender aufgebrachten mechanischen Arbeit beim Öffnen oder Schließen der Andruckeinrichtung zwischenzuspeichern und dann ein "arbeitsreduziertes" Schließen oder Öffnen der Andruckeinrichtung zu ermöglichen. In jedem Fall ist es aber der Anwender, der die mechanische Arbeit aufwenden muß.

Aufgabe der Erfindung ist es daher, einen Druckinfusionsapparat derart zu verbessern, daß die notwendige Arbeit zum Öffnen oder Schließen der Andruckeinrichtung nicht durch den Anwender, sondern durch einen innerhalb oder außerhalb aufgebrachten Öffnungsmechanismus aufgebracht wird, so daß der Druckinfusionsapparat auch vom geriatrischen und/oder morbiden Anwender benutzt werden kann.

Diese Aufgabe wird dadurch gelöst, daß zum selbsttätigen Öffnen bzw. Schließen der Andrückeinrichtung zwischen der Fördermechanik und der Andrückeinrichtung eine Kurvenscheibe angeordnet ist, die mit einem Abschnitt ihres Außenumfanges an der Andrückeinrichtung anliegt, die unter der Wirkung einer Kraft gegen die Kurvenscheibe drückt, und die über drehrichtungsabhängige Mitnehmer mit der Fördermechanik verbunden ist, so daß bei einer vorgegebenen Drehrichtung für den Antrieb der Fördermechanik deren andere Drehrichtung derart nutzbar ist, daß die Kurvenscheibe die Andrückeinrichtung für den Schlauch in Abhängigkeit vom Drehwinkel von der Fördermechanik wegdrücken kann.

Zur Förderung der Infusionsflüssigkeit wird in dem Druckinfusionsapparat ein Antrieb benutzt, der meist nur in einer Drehrichtung benötigt wird. Die andere Drehrichtung ist frei und wird erfindungsgemäß zur Betätigung des Öffnungsmechanismus herangezogen werden. Durch die Benutzung des bereits vorhandenen Antriebs braucht der Anwender den Öffnungs- und Schließvorgang nur durch Knopfdruck anzusteuern, wodurch lediglich ein Bruchteil der Arbeit notwendig wird, die für das direkte Öffnen oder Schließen der Andrückeinrichtung gebraucht wird. Durch den reduzierten Arbeitsbedarf für das Öffnung und Schließen wird eine bessere Handhabung für den geriatrischen und/oder morbiden Anwender erreicht.

Die Funktion des erfindungsgemäßen Druckinfusionsapparates beruht darauf, daß die Kurvenscheibe die Andrückeinrichtung für den Schlauch in Abhängigkeit vom Drehwinkel von der Fördermechanik weg bzw. an die Fördermechanik drücken kann. Die kraftschlüssige Berührung zwischen der Andrückeinrichtung und der Kurvenscheibe wird durch eine gegen die Andrückeinrichtung gerichtete Kraft gewährleistet, die beispielsweise durch eine Feder aufgebracht werden kann. Durch mindestens einen drehrichtungsabhängigen Mitnehmer wird die Kurvenscheibe dann mit der Fördereinrichtung gekoppelt, wenn sich die Fördereinrichtung entgegen der Förderrichtung bewegt. Die notwendige Energie zum Öffnen der Andruckeinrichtung wird hierbei von der Motorgetriebeeinheit der Pumpe aufgebracht. Durch bekannte elektronische oder mechanische Maßnahmen ist es möglich, daß der Anwender den Stator nur öffnen kann, wenn die Pumpe nicht auf Förderung geschaltet ist.

Gemäß einer besonderen Ausführungsform ist die Kurvenscheibe kreisförmig ausgebildet und ihr Drehpunkt exzentrisch angeordnet. Bei einem Druckinfusionsapparat mit Rotor und Stator ist die Kurvenscheibe unter dem Rotor angeordnet, wobei der Drehpunkt der Kurvenscheibe und der Drehpunkt des Rotors zusammenfallen.

Damit das "Öffnen" und "Schließen" automatisierbar gemacht werden kann, ist eine Positionserkennung der Kurvenscheibe erforderlich. Dies wird gemäß einer Ausführungsform dadurch erreicht, daß in oder an der Kurvenscheibe ein Magnet angeordnet ist, der mit mindestens einem Hall-Sensor zusammenwirkt, der beispielsweise am Gehäuse bei einer bestimmten Winkelstellung der Kurvenscheibe angebracht ist. Vorzugsweise werden zwei Hall-Sensoren bei der 0° und und der 180°-Stellung der Kurvenscheibe angeordnet, die die Offenstellung bzw. die Schließstellung markieren. Die Drehrichtung, die zum Öffnen und Schließen der Andruckeinrichtung führt, ist entgegengesetzt der Drehrichtung, die zum Fördern der Infusionsflüssigkeit benutzt wird.

Als Mitnehmer können unterschiedliche Bauteile zum Einsatz kommen.

So können die Mitnehmer aus mindestens einer Zahnklinke bestehen, die an ihrem einen Ende an der Kurvenscheibe oder der Fördermechanik befestigt ist. Wird die Drehrichtung der Fördermechanik geändert, also von "Fördern" auf "Öffnen", greift die Zahnklinke mit ihrem anderen Ende in eine entsprechende Ausnehmung in der Fördermechanik bzw. in der Kurvenscheibe.

Gemäß einer anderen Ausführungsform bestehen die Mitnehmer aus zwei Textilstreifen, die mit ihrem Flor aneinanderliegen. Die Florausrichtung der beiden Textilstreifen ist entgegengesetzt, so daß die beiden Flore nur bei einer Bewegungsrichtung ineinandergreifen und so eine kraftschlüssige Verbindung zwischen Fördermechanik und Andruckeinrichtung herstellen.

Gemäß einer weiteren Ausführungsform bestehen die Mitnehmer aus mindestens einer Spiralfeder, die mit ihrem einen Ende an der Kurvenscheibe oder der Fördermechanik und bei vorgegebener Drehrichtung mit ihrem anderen Ende in eine entsprechende Ausnehmung in der Fördermechanik oder in der Kurvenscheibe eingreift.

Der Antrieb des Druckinfusionsapparates ist derart gesteuert und ausgebildet, daß das Öffnen und Schließen automatisch abläuft.

Nachfolgend werden beispielhafte Ausführungsformen der Erfindung anhand der Zeichnung näher erläutert.

Es zeigen:
- Figur 1: eine Draufsicht auf Kurvenscheibe und Stator,
- Figur 2: einen Schnitt längs der Linie I-I der in Figur 1 gezeigten Ausführungsform,
- Figur 3: eine weitere Ausführungsform bei einer peristaltischen Pumpe,
- Figur 4: eine weitere Ausführungsform bei einer statorlosen Pumpe,
- Figur 5a, b, c: drei Ausführungsformen der Mitnehmer.

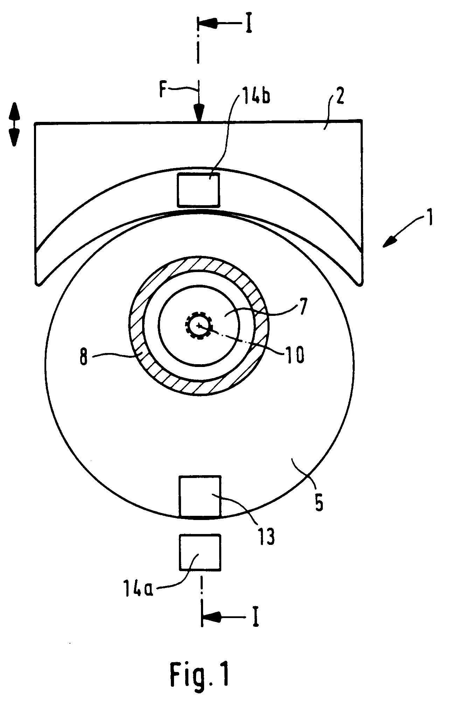

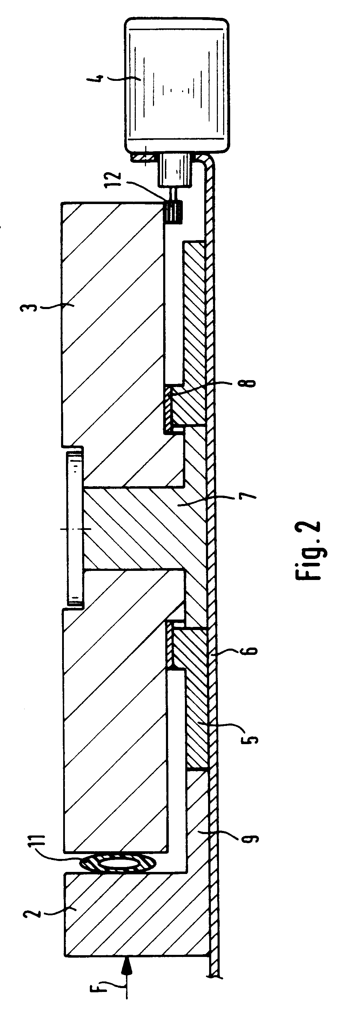

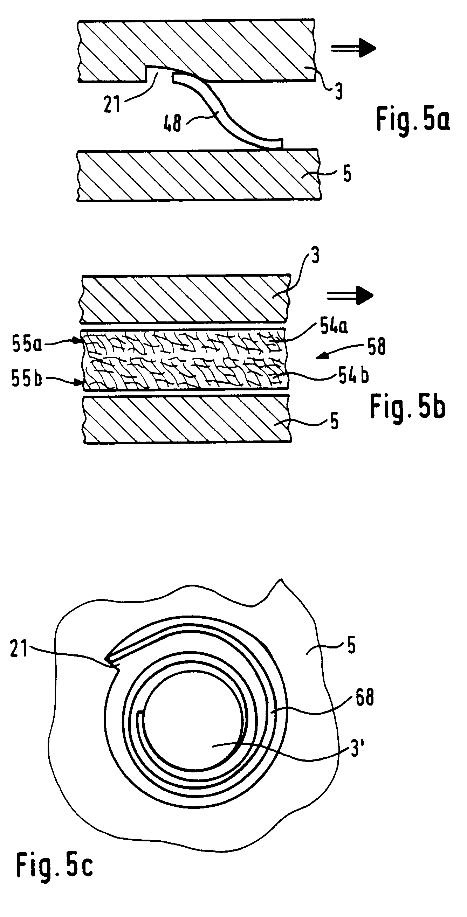

In Figur 1 ist schematisch ein Druckinfusionsapparat 1 mit Rotor 3 (s. Figur 2) und Stator 2 dargestellt. Die Kurvenscheibe 5 besitzt einen exzentrisch angeordneten Drehpunkt 10, der mit dem Drehpunkt des Rotors 3 zusammenfällt. Die Kurvenscheibe 5 ist mittels des Lagerbocks 7 drehbar gelagert. An der Oberseite der Kurvenscheibe ist schematisch ein drehrichtungsabhängiger Mitnehmer 8 eingezeichnet, der nachfolgend in den Figuren 5a, b und c noch ausführlicher beschrieben wird. Die Kurvenscheibe 5 liegt mit einem Abschnitt ihres Außenumfanges an dem Stator 2 an, der mit einer Kraft F gegen die Kurvenscheibe gedrückt wird. Diese Kraft F kann durch eine nichtdargestellte Feder aufgebracht werden.

Die Kurvenscheibe 5 ist mit einem magnetischen Bereich 13 versehen, der zur Positionserkennung der Kurvenscheibe mit zwei Hallsensoren 14a und 14b zusammenwirkt. Die in Figur 1 gezeigte Position der Kurvenscheibe ist die Schließstellung. Wird die Kurvenscheibe um 180°C weitergedreht, ist die Offenstellung erreicht, die dann vom Sensor 14b registriert wird, der den Antrieb abschaltet. Soll nach dem Einlegen des Schlauches 11 (s. Fig. 2) der Stator in Schließstellung gebracht werden, wird bei gleicher Drehrichtung wie zuvor die Kurvenscheibe wiederum um 180°C weiterbewegt, bis der Magnet 13 dem Sensor 14a gegenüberliegt, der dann den Antrieb 4 auf "Fördern", also in entgegensetzter Drehrichtung umschaltet. Bei entgegengesetzter Drehrichtung gelangen Rotor 3 und Kurvenscheibe 5 durch die Ausgestaltung der Mitnehmer 8 außer Eingriff, so daß während der Förderung die Kurvenscheibe 5 in Ruhe bleibt.

In Figur 2 ist ein Schnitt längs der Linie I-I der in Figur 1 gezeigten Ausführungsform dargestellt. Auf einer gemeinsamen Grundplatte 6 ist der Stator 2 verschiebbar gegen den Rotor 3 bzw. die Kurvenscheibe 5 angeordnet. Der Rotor besitzt einen Ansatz 9, der unter den Rotor 3 ragt und an der Kurvenscheibe 5 anliegt. Zwischen Stator 2 und Rotor 3 ist ein zusammengequetschter Schlauch 11 eingezeichnet (Schließstellung). Der Rotor 3 und die Kurvenscheibe 5 sind mittels des Lagerbocks 7 drehbar angeordnet. Der Rotor 3 wird über ein Zahnrad 12 von einem Antrieb 4, der auch ein Getriebe aufweist, angetrieben. An der Oberseite der Kurvenscheibe 5 ist ein drehrichtungsabhängiger Mitnehmer 8 angeordnet, der entsprechend einer vorgegebenen Drehrichtung des Rotors 3 die Kurvenscheibe 5 in Drehrichtung versetzt, so daß der Stator geöffnet bzw. geschlossen wird.

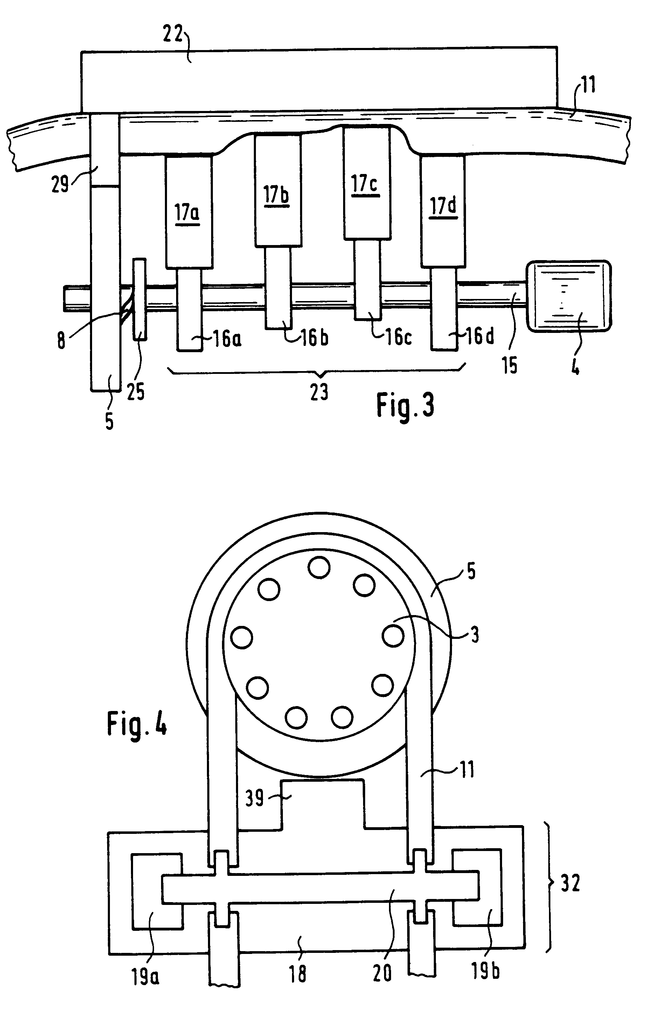

In der Figur 3 ist schematisch ein Druckinfusionsapparat mit einer Fingerperistaltik dargestellt. Die Fördermechanik 23 besteht in dem hier gezeigten Beispiel aus vier Pumpstößeln 17a bis 17d, die über entsprechende Kurvenscheiben 16a bis 16d mit einer Antriebswelle 15 verbunden sind. Durch die Anordnung und Ausgestaltung der Kurvenscheiben 16a bis 16d führen die Pumpstößel 17a bis 17d eine peristaltische Bewegung aus, durch die die Flüssigkeit im Schlauch 11 gefördert wird. Die Andruckeinrichtung, die den Pumpstößeln 17a bis 17d gegenüberliegt, ist in dem hier gezeigten Beispiel eine Andruckplatte 22, die einen Ansatz 29 aufweist, der an einem Abschnitt des Außenumfangs der Kurvenscheibe 5 anliegt. Die Kurvenscheibe 5 kann wie in den Ausführungsbeispielen der Figuren 1 und 2 ebenfall kreisförmig ausgebildet sein, wobei der Drehpunkt exzentrisch angeordnet ist. Die Kurvenscheibe 5 ist auf der Antriebswelle 15 drehbar gelagert und steht mit der Fördermechanik 23 über einem Mitnehmer 8 und einer weiteren Platte 25, die fest auf der Antriebswelle 5 befestigt ist, in Verbindung.

Wenn die Antriebswelle 15 sich in Förderrichtung bewegt, d.h. die Pumpstößel 17a bis 17d führen eine peristaltische Bewegung aus, dann steht die Kurvenscheibe 5 nicht in Eingriff mit der Fördermechanik 23. Wenn jedoch die Drehrichtung der Antriebswelle 15 geändert wird, greift der Mitnehmer 8, der in diesem Fall als Zahnklinke ausgebildet ist, in eine entsprechende Ausnehmung in der Kurvenscheibe 5 und dreht diese in gleicher Richtung. Dadurch wird die Andruckplatte 22 von der Fördermechanik 23 wegbewegt, so daß der Schlauch 11 freigegeben wird und aus dem Druckinfusionsapparat herausgenommen werden kann. Nach dem Auswechseln des Schlauches 11 wird die Antriebswelle 15 in gleicher Richtung weitergedreht, was zur Folge hat, daß auch die Kurvenscheibe in gleicher Richtung weitergedreht wird. Dadurch wird die Andruckscheibe 22 wieder in Schließstellung gebracht.

In Figur 4 ist schematisch eine statorlose Pumpe dargestellt. Diese statorlose Pumpe weist einen Rotor 3 auf, der eine Anzahl von Rollen 27 enthält, die kreisförmig angeordnet sind. Der Schlauch 11 ist zu einer Schlaufe gebogen und an einer Schlauchplatte 20 befestigt. Unter dem Rotor 3 ist ähnlich der Ausführungsform der Figur 2 die Kurvenscheibe 5 angeordnet, die mit einem Abschnitt ihres Außenumfangs an einem Ansatz 39 der Andruckplatte 18 anliegt. Auf der Oberseite der Andruckplatte 18 sind zwei Halterungen 19a und 19b befestigt, in die die Schlauchplatte 20 eingesteckt wird.

Zum Einlegen des Schlauches 11 wird die Andruckeinrichtung 32 mittels der Kurvenscheibe 5 auf den Rotor 3 zubewegt. Das Schlauchsegment 11 kann dann über den Rotor gelegt werden und die Schlauchplatte 20 kann ohne zusätzliche Kraftaufwendung in die Halterungen 19a und 19b eingesteckt werden. Anschließend wird die Kurvenscheibe 5 weitergedreht, was zur Folge hat, daß die Andruckeinrichtung 32 vom Rotor 3 wegbewegt wird. Dadurch wird der Schlauch 11 gespannt und die Rollen 27 drücken den Schlauch im oberen Abschnitt soweit zusammen, daß bei Drehung des Rotors 3 in Förderrichtung die in dem Schlauch 11 befindliche Flüssigkeit gefördert wird. Bei derartigen Pumpen wird im Gegensatz zu den vorher beschriebenen Druckinfusionsapparaten die Offenstellung durch ein Aufeinanderzubewegen von Rotor und Andruckeinrichtung und die Schließstellung durch ein Voneinanderwegbewegen erreicht.

In den Figuren 5a, b und c sind drei Ausführungsformen der Mitnehmer 8 dargestellt. In der Figur 5a besteht der Mitnehmer 8 aus einer Zahnklinke 48, die mit einem Ende auf der Kurvenscheibe 5 befestigt ist. Mit ihrem anderen Ende greift der Mitnehmer 48 in eine Ausnehmung 21, wenn sich der Rotor 3 in Pfeilrichtung bewegt. In diesem Fall wird die Kurvenscheibe 5 in dieser Richtung mitgenommen. Wenn sich der Rotor 3 entgegengesetzt der Pfeilrichtung bewegt, gelangt die Zahnklinke 48 außer Eingriff mit dem Rotor 3, so daß die Kurvenscheibe 5 nicht mitbewegt wird.

In der Figur 5b bestehen die Mitnehmer 58 aus zwei Textilstreifen 55a und 55b. Die Flore sind durch die Bezugszeichen 54a und 54b gekennzeichnet. Die Flore 54a und 54b sind entgegengesetzt ausgerichtet, so daß bei einer Bewegung des Rotors 3 in Pfeinrichtung die Kurvenscheibe 5 mitgenommen wird.

In der Figur 5c besteht der Mitnehmer 8 aus einer Spiralfeder 68, die mit ihrem einen Ende an den Zapfen 3′ des Rotors 3 befestigt ist. Mit dem anderen Ende greift die Spiralfeder in eine entsprechende Ausnehmung 21 der Kurvenscheibe 5. Die Funktionsweise entspricht der in Figur 5a dargestellten Ausführungsform.

## Patentansprüche

1. Druckinfusionsapparat mit einem Antrieb, der über eine Fördermechanik (3, 23), wie Rotor oder Fingerperistaltik, eine während der Förderung periodische, fortschreitende Druckbewegung auf einen Abschnitt eines Schlauches (11) ausübt, in dem sich das zu fördernde Medium befindet, und mit einer während der Förderung feststehenden Andrückeinrichtung (2, 22, 32), Andrückplatte, Stator oder Schlauchplatte, dadurch gekennzeichnet,
daß zum selbstätigen Öffnen bzw. Schließen der Andrückeinrichtung zwischen der Fördermechanik (3, 23) und der Andrückeinrichtung (2, 22, 32) eine Kurvenscheibe (5) angeordnet ist, die mit einem Abschnitt ihres Außenumfangs an der Andrückeinrichtung (2, 22, 32) anliegt, die unter der Wirkung einer Kraft gegen die Kurvenscheibe (5) drückt, und die über drehrichtungsabhängige Mitnehmer (8, 58, 68) mit der Fördermechanik (3, 23) verbunden ist, so daß bei einer vorgegebenen Drehrichtung für den Antrieb der Fördermechanik deren andere Drehrichtung derart unterbar ist, daß die Kurvenschiebe die Andrückeinrichtung (2, 22, 32) für den Schlauch in Abhängigkeit vom Drehwinkel von der Fördermechanik wegdrücken kann.

2. Druckinfusionsapparat nach Anspruch 1, dadurch gekennzeichnet, daß die Kurvenscheibe (5) kreisförmig ausgebildet ist und der Drehpunkt (10) exzentrisch angeordnet ist.

3. Druckinfusionsapparat nach Anspruch 1 oder 2, dadurch gekennzeichnet, daß zur Positionserkennung die Kurvenscheibe (5) einen magnetischen Bereich (13) aufweist, der mit mindestens einem Hall-Sensor (14a, 14b) zusammenwirkt.

4. Druckinfusionsapparat nach einem der Ansprüche 1 bis 3, dadurch gekennzeichnet, daß die Mitnehmer aus mindestens einer Zahnklinke (48) bestehen, die an ihrem einen Ende an der Kurvenscheibe (5) oder der Fördermechanik (3, 23) befestigt ist und bei vorgegebener Drehrichtung mit ihrem anderen Ende in eine entsprechende Ausnehmung (21) in der Fördermechanik (3, 23) oder in der Kurvenscheibe (5) eingreift.

5. Druckinfusionsapparat nach einem der Ansprüche 1 bis 3, dadurch gekennzeichnet, daß die Mitnehmer aus zwei Textilstreifen (55a, 55b) mit jeweils entgegengesetzter Ausrichtung des Flors (54a, 54b) bestehen, wovon ein Textilstreifen (55a oder 55b) auf der Kurvenscheibe (5) und ein Textilstreifen (55b oder 55a) an der Fördermechanik (3, 23) befestigt ist.

6. Druckinfusionsapparat nach einem der Ansprüche 1 bis 3, dadurch gekennzeichnet, daß die Mitnehmer aus mindestens einer Spiralfeder (68) bestehen, die mit ihrem einen Ende an der Kurvenscheibe (5) oder der Fördermechanik (3, 23) befestigt ist und bei vorgegebener Drehrichtung mit ihrem anderen Ende in eine entsprechende Ausnehmung (21) in der Fördermechanik (3, 23) oder in der Kurvenscheibe (5) eingreift.

## Claims

1. Pressure infusion apparatus comprising a drive which exerts, via a delivery mechanism (3,23), such as rotor or finger peristaltic, an advancing periodic pressure movement during delivery on a section of a flexible tube (11) containing the medium to be delivered, and a pressure means (2, 22, 32) such as pressure plate, stator or tube plate which is stationary during delivery, characterized in that for automatic opening, resp closing of said pressure plate a cam plate (5) is disposed between the delivery mechanism (3, 23) and the pressure means (2, 22, 32), a portion of the outer periphery of said cam plate (5) bearing on the pressure means (2, 22, 32) which under the action of a force presses against the cam plate (5) and which is connected to the delivery mechanism (3, 23) by means of a rotation direction-dependent driver (8, 58, 68) such that with a predetermined direction of rotation for the drive of the delivery mechanism its other direction of rotation is utilizable such that the cam plate (5) can press the pressure means (2, 22, 32) for the tube in dependence from the angle of rotation away from the delivery mechanism.

2. Pressure infusion apparatus according to claim 1, characterized in that the cam plate (5) is circular and comprises an eccentrically disposed rotation center (10).

3. Pressure infusion apparatus according to claim 1 or 2, characterized in that the cam plate (5) comprises a magnetic region (13) which cooperates with at least one Hall sensor (14a, 14b) for position detection.

4. Pressure infusion apparatus according to any of claims 1-3, characterized in that the drivers comprise at least one pawl (48) which is secured at a first end thereof to the cam plate (5) or the delivery mechanism (3,23) and at a given direction of rotation engages at a second end thereof into a corresponding recess (21) in the delivery mechanism (2, 23) or in the cam plate (5).

5. Pressure infusion apparatus according to any of claims 1-3, characterized in that the drivers comprise two textile strips (55a, 55b) each having oppositely aligned pile (54a, 54b), one textile strip (55a or 55b) being secured to the cam plate (5) and one textile strip (55b or 55a) being secured to the delivery mechanism (3, 23).

6. Pressure infusion apparatus according to any of claims 1-3, characterized in that the drivers comprise at least one spiral spring (68) which is secured at a first end thereof to the cam plate (5) or the delivery mechanism (3, 23) and at a given direction of rotation engages at a second end thereof into a corresponding recess (21) in the delivery mechanism (3, 23) or in the cam plate (5).

## Revendications

1. Dispositif de perfusion sous pression comportant un dispositif d'entraînement, qui, par l'intermédiaire d'un système mécanique de refoulement (3,23), comme le rotor ou un système péristaltique à doigts, provoque, dans une section d'appui (11), dans laquelle est situé le milieu à entraîner, un déplacement continu en compression, qui est périodique pendant le refoulement, et comportant un dispositif de serrage (2,22,32), comme par exemple une plaque de serrage, un stator et une plaque porte-tuyau, qui est fixe pendant le refoulement, caractérisé en ce que pour l'ouverture et la fermeture automatique du dispositif de serrage, entre le système mécanique de refoulement (3,23) et le dispositif de serrage (2,22,32) est disposé un disque formant came (25), qui s'applique au moyen d'une partie de sa circonférence extérieure, contre le dispositif de serrage (2,22,32) qui s'applique sous l'action d'une force contre le disque formant came (5) et qui est raccordé, par l'intermédiaire d'organes d'entraînement (8,58,68), qui agissent en fonction du sens de rotation, au système mécanique de refoulement (3,23) de sorte que, un sens de rotation prédéterminé étant prévu pour l'entraînement du système mécanique de refoulement, l'autre sens de rotation peut être utilisé de telle sorte que le disque formant came peut écarter du système mécanique de refoulement, le dispositif de serrage (2,22,32) pour le tuyau, en fonction de l'angle de rotation.

2. Dispositif de perfusion sous pression selon la revendication 1, caractérisé en ce que le disque formant came (5) est agencé avec une forme circulaire et le centre de rotation (10) est excentré.

3. Dispositif de perfusion sous pression selon la revendication 1 ou 2, caractérisé en ce que pour l'identification de la position, le disque formant came (5) comporte une partie magnétique (13), qui coopère avec au moins un capteur de Hall (14a,14b).

4. Dispositif de perfusion sous pression suivant l'une des revendications 1 à 3, caractérisé par le fait que les organes d'entraînement sont constitués par au moins un cliquet (41), qui est fixé, par l'une de ses deux extrémités, au disque formant came (5) ou au système mécanique de refoulement (3,23) et, dans un sens de rotation prédéterminé, s'engage, par son autre extrémité, dans un évidement correspondant (21) ménagé dans le système mécanique de refoulement (3,23) ou dans le disque formant came (5).

5. Dispositif de perfusion sous pression selon l'une des revendications 1 à 3, caractérisé en ce que les organes d'entraînement sont constitués par deux bandes textiles (55a, 55b) dont les nappes de poils (54a,54b) sont orientées respectivement en des sens opposés, et parmi lesquels une bande textile (55a ou 55b) est fixée sur le disque formant came (5) et une bande textile (55b ou 55a) est fixée sur le système mécanique de refoulement (3,23).

6. Dispositif de perfusion sous pression selon l'une des revendications 1 à 3, caractérisé en ce que les organes d'entraînement sont constitués par au moins un ressort spiral (68), dont l'une des extrémités est fixée au disque formant came (55) ou au système mécanique de refoulement (3,23) et dont l'autre extrémité s'engage, dans un sens de rotation prédéterminé, dans un évidement correspondant (29) ménagé dans le système mécanique de refoulement (3,23) ou dans le disque formant came (5).
